# EUROPEAN PATENT APPLICATION

(11) **EP 1 481 656 A1**
(43) Date of publication of application: **01.12.2004**
(21) Application number: 03447125.0
(22) Date of filing: 26.05.2003
(51) Int. Cl.: A61F 13/20

(54) **Tampon having differentiated expansion capacity**

(71) Applicant: Ontex N.V., 9255 Buggenhount (BE)
(72) Inventor: Van Ingelgem, Werner, 9255 Buggenhout (BE); De Geest, Annelies, 9310 Baardegem (BE); Mahlous, Djamila, 1000 Brussel (BE)
(74) Representative: Brants, Johan Philippe Emile

(57) **Abstract**

The present invention relates to a tampon (5) for feminine hygiene having a longitudinal body (2) provided with a round domed insertion end (1) and a withdrawal end (3), from which withdrawal end (3) a withdrawal cord (4) extends, whereby the tampon (5) essentially consists of compressed absorbent fibrous material and optionally comprises longitudinal grooves (6), whereby said tampon (5) comprises differentiated expansion capacity in the longitudinal direction, decreasing towards the withdrawal area.

## Description

### Field of the invention

The present invention relates to a tampon for feminine hygiene and to a method for producing said tampon.

### Background of the invention

Intravaginal tampons are in common use by women for the retention of fluids or menses discharged along the walls of the vagina during the menstrual cycle. The menstrual discharge, comprising endometrial cells, secretions and blood, is intermittent and takes place over hours and days. The blood and other matter exude following the line of gravity. Sometimes the flow is light, sometimes heavy. Intravaginal tampons are usually formed of absorbent materials such as cotton, rayon cellulose wading, synthetic sponge, cellulose fluff, synthetic fibres or combinations of these materials and compressed or molded usually to a generally cylindrical configuration of a size to fit within the vaginal tract.

Tampons having an insertion end, a withdrawal end, a withdrawal cord and a central section extending therebetween are well known in the art.

The use of tampons can be accompanied with undesirable irritation as a result of insertion and withdrawal, especially during periods of light menses flow or when the cavity is relatively dry. The tendency of the tampon to absorb whatever small amount of liquids are present on the vaginal walls generates excess friction between the tampon and the vaginal walls. Insertion and withdrawal of the tampon may therefore become painful.

It is therefore a general object of the present invention to provide a tampon being more convenient and comfortable for use. The present invention also aims to provide a tampon, which avoids menses to be absorbed by the withdrawal cord.

### Summary of the invention

The current invention provides a tampon, which overcomes at least some of the above-mentioned drawbacks of currently available tampons.

In a first aspect, the present invention relates to a tampon for feminine hygiene having a longitudinal body provided with a round domed insertion end and a withdrawal end, from which withdrawal end a withdrawal cord extends, whereby the tampon essentially consists of compressed absorbent fibrous material and optionally comprises longitudinal grooves, whereby said tampon comprises differentiated expansion capacity in the longitudinal direction, decreasing towards the withdrawal end.

In another aspect, the present invention relates to a tampon for feminine hygiene having a longitudinal body provided with a round domed insertion end and a withdrawal end, from which withdrawal end a withdrawal cord extends, and whereby the tampon essentially consists of compressed absorbent fibrous material and whereby said tampon comprises longitudinal grooves and whereby said tampon has a constricted withdrawal end.

The present invention provides a tampon, wherein the insertion end has a round domed shape and is provided with a round dome on the top. Such tampon is easy to insert because a narrowed end goes deepest in the vagina. The tampon is further provided with longitudinal grooves which bring about an enlargement of the surface of the tampon and which consequently considerably improve the absorption capacity and expansion capacity of the tampon. The structure of the tampon is chosen in such a manner that the withdrawal of the expanded tampon out of the body cavity is facilitated. Faster expansion of the insertion end of the tampon, improved absorption of liquid in the insertion end and/or reducing expansion of the withdrawal end have the advantage that the tampon becomes smaller towards the withdrawal end. This makes withdrawal easier and less painful, especially when menstruation is limited, e.g. in beginning and at the end of the menstrual period.

In one aspect, the tampon comprises a constricted withdrawal end. A "constricted withdrawal end", as used herein, refers to a withdrawal end which has a cross sectional diameter which is smaller than the cross-sectional diameter of the remaining tampon body. In a preferred embodiment, the invention provides a tampon, wherein said withdrawal end has a frusto-conical shape. The withdrawal end of the tampon can be further constructed so as to expand less and considerably facilitate withdrawal of the tampon, especially when the tampon is not saturated.

In particular, the present invention provides a tampon having improved expansion and absorptive capacity in and or in the vicinity of the insertion end of the tampon compared with the remaining tampon body. A higher expansion capacity in the upper portion of the tampon may be obtained by various technical features. The term "expansion" as used herein is in certain cases used as a synonym for "absorption". It should be understood that the term "expansion" relates to the absorbance capacity and the mechanical features of the absorbent material used in the tampons of the present invention.

A tampon, which expands more in and or in the vicinity of the insertion end than in the remaining body of the tampon according to the invention, induces faster absorption and facilitates liquid uptake in the upper portion of the tampon. This area will therefore be able to absorb menses faster and thus in practice to absorb more menses. It must be realised that because the insertion end is located near the origin of the menses, i.e., the cervix, the menses will be concentrated more heavily in the insertion end than in other areas of the tampon. Therefore, it is important that the upper portion of the tampon is able to absorb faster and thus more menses than the rest of the tampon body. Also, when the tampon has just been inserted, the upper portion will expand faster and the risk of by-pass and leakage can be considerably reduced. The present invention thus also provides a tampon, which considerably reduces risk of leakage after the tampon has been put into use.

In another aspect, the invention relates to a method for fabricating a tampon according to the invention. The method for producing a tampon comprises the steps of providing a tampon blank of tangled fibrous material having a longitudinal direction; and compressing the tampon blank whereby longitudinal grooves and longitudinal ribs are at least partially formed at an outer surface of the tampon, and whereby the fibrous material in the area of at least the insertion end and the withdrawal end of the tampon blank is subjected to a higher radial compressing pressure than the remaining fibrous material of the tampon blank.

Those skilled in the art will immediately recognise the many advantages of the present invention from the detailed description and accompanying figures provided below.

### Detailed description of the figures

Figures 1, 2, 3 and 4 show different embodiments of a tampon according to the invention before use.

Figure 5 illustrates an embodiment of a tampon according to the invention prior to use.

Figures 6 and 7 represent two embodiments of a tampon as illustrated in figure 5 in expanded condition.

Figure 8 illustrates the use of a tampon according to the invention. In Fig. 8A the tampon is inserted in the vagina. By absorbing menses, the tampon will expand as illustrated in Fig. 8B.

Fig. 8C illustrates withdrawal of the tampon from the vagina.

### Detailed description of the invention

Tampons having an insertion end, a withdrawal end, a withdrawal cord and a central section extending therebetween are well known in the art. However, several problems are associated with the use of intravaginal tampons for the collection and retention of menstrual fluids. In general, tampons generally expand substantially uniformly over their total length when engaged by fluid. If the fluid is not uniformly or only partially applied to the tampon, increased fluid concentrations are developed in certain regions of the tampon. Lack of sufficient absorption of the tampon in these regions may lead to undesirable leakage. Also, the peripheral interior contour of the vaginal wall, being unpredictably irregular as compared with the preformed tampon, often leads to the by-pass of fluid menses through the occasional spaces encountered between the outer surface of the tampon and the inner vaginal wall. Bypass failure occurs when the menses travels the length of the vagina without contacting the tampon, i.e., the tampon fails to intercept the flowing menses.

Another disadvantage associated with the use of state of the art intravaginal tampons includes partitioning failure. Partitioning failure occurs when the menses flow rate past a particular area of the tampon is greater than the absorption rate into the tampon in that area. Thus, although some of the menses is absorbed, that flow which is greater than the absorption rate into the tampon proceeds past the tampon and out the body.

Another problem is that after use, currently available tampons have a withdrawal end which has expanded at least as much as the rest of the tampon body. This makes withdrawal more difficult, especially when menstruation is limited, e.g. in beginning and at the end of the menstrual period.

Another important problem associated with currently available tampons is their lack of absorptive capacity. State of the art tampons have inadequate absorptive capacity, which leads to inconveniences such as leakage and/or by pass problems. This lack of absorptive capacity may be due to the fact that when a tampon is inserted, it does not start absorbing fast enough to avoid by-pass and leakage. Furthermore, in some cases the currently known tampons having insufficient absorption capacity have the effect that menses can be absorbed by the withdrawal cord, which is particularly undesirable from a hygienical point of view. Such absorption by the withdrawal cord also results in stains in underpants and a lack of faith of a customer in the absorbent capacity of the tampon. On the other hand, tampons having sufficient absorptive capacity are generally voluminous. However, voluminous tampons, especially those with a high absorption capacity, lack comfort for insertion and, especially if the tampon is not saturated, for withdrawal.

The present invention provides a tampon, which may overcome at least some of the above-mentioned disadvantages of currently known tampons. In a first embodiment the present invention relates to a tampon for feminine hygiene having a longitudinal body provided with a round domed insertion end 1 and a withdrawal end 3, from which withdrawal end 3 a withdrawal cord 4 extends, whereby the tampon essentially consists of compressed absorbent fibrous material and optionally comprises longitudinal grooves 6, whereby said tampon comprises differentiated expansion capacity in the longitudinal direction, decreasing towards the withdrawal end 3.

In a second embodiment, the present invention relates to a tampon for feminine hygiene having a longitudinal body 2, a round domed insertion end 1 and a withdrawal end 3, from which withdrawal end 3 a withdrawal cord 4 extends, and whereby the tampon essentially consists of compressed absorbent fibrous material and whereby said tampon comprises longitudinal grooves 6 and whereby said tampon has a constricted withdrawal end 3.

In yet another aspect, the present invention relates to a tampon for feminine hygiene having a longitudinal body 2, a round domed insertion end 1 and a withdrawal end 3, from which withdrawal end 3 a withdrawal cord 4 extends, and whereby the tampon essentially consists of compressed absorbent fibrous material, whereby said tampon comprises differentiated expansion capacity in the longitudinal direction, decreasing towards the withdrawal end 3, and whereby said tampon has a constricted withdrawal end 3 and comprises longitudinal grooves 6.

Absorbent fibrous material usable in the tampon according to the invention may consist of any absorbent material having acceptable absorbency and modulus of elasticity properties that is capable of absorbing and/or retaining liquid. The absorbent structure can be manufactured in a wide variety of sizes and shapes and from a wide variety of liquid-absorbing materials. It is, of course, desirable to use absorbent materials having a minimum content of extraneous soluble materials since the product may be retained in the body for a considerable period of time. Retained soluble extraneous materials could cause a safety hazard if they are toxic, irritant, or sensitive. A representative, non-limiting list of useful materials includes cellulosic materials, such as rayon, cotton, wood pulp, creped cellulose wadding, tissue wraps and laminates, peat moss, and chemically stiffened, modified, or cross-linked cellulosic fibres; synthetic materials, such as polyester fibres, polyolefin fibres, absorbent foams, e.g. a flexible resilient polyurethane foam, absorbent sponges, superabsorbent polymers, absorbent gelling materials; formed fibres, such as capillary channel fibres and multi limbed fibres; synthetic fibres, or any equivalent material or combinations of materials, or mixtures of these.

In a preferred embodiment, the absorbing capacity of the present tampon is further improved by selecting more absorptive material. When using material that absorbs more, the tampons are less voluminous, thus facilitating insertion and, especially when the tampon is not saturated, withdrawal of the tampon. Therefore, fibrous material usable in the tampon according to the invention preferably comprises more absorptive materials such as e.g. open-celled foam. The use of more absorptive material will also further reduce the risk of leakage and by-pass.

Furthermore, the present invention relates to tampons, which can be applied digitally, as well as to tampons that can be applied with an inserter and an ejector. An inserter and an ejector used to eject the tampon from the inserter after the inserter is positioned within the vagina can be any inserter known to those skilled in the art, e.g., the telescoping tube type inserters. The inserter and the ejector can be made of any of the acceptable materials, e.g., cardboard or molded polyethylene. The inserter can be sized similarly to those presently commercially used.

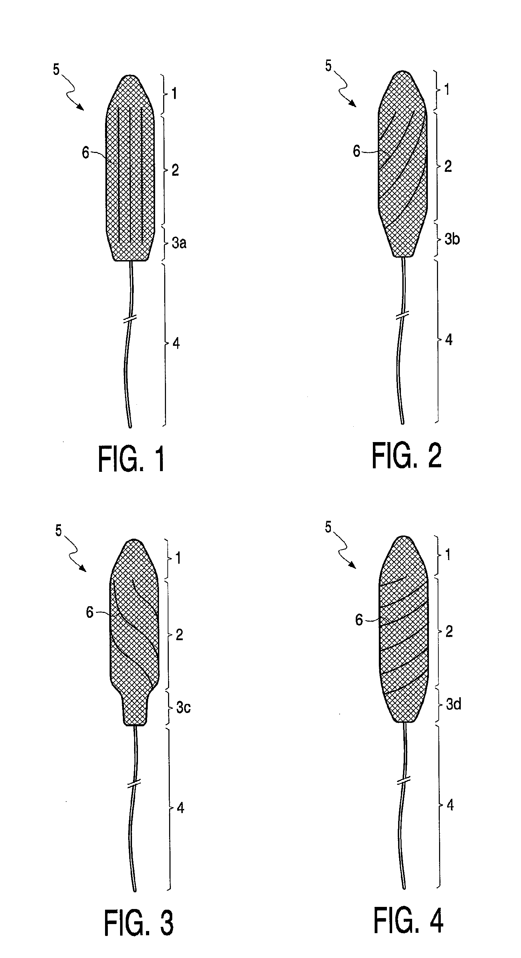

According to one embodiment of the present invention, **FIG. 1** illustrates a tampon for feminine hygiene, which is made of compressed absorbent fibrous material. The tampon 5 comprises a longitudinal body 2 provided with an insertion end 1 and a withdrawal end 3. Further preferred embodiments of a tampon according to the present invention are illustrated in **FIG. 1-4**. The tampons 5 illustrated in **FIG. 1-4**, all have an insertion end 1, which is preferably dome shaped, and a withdrawal end 3a, 3b, 3c, 3d, which is preferably constricted. The illustrated tampons 5 are preferably provided with longitudinal grooves 6, which may be straight, as illustrated in **FIG. 1** or spirally shaped, as illustrated in **FIG. 2-4**.

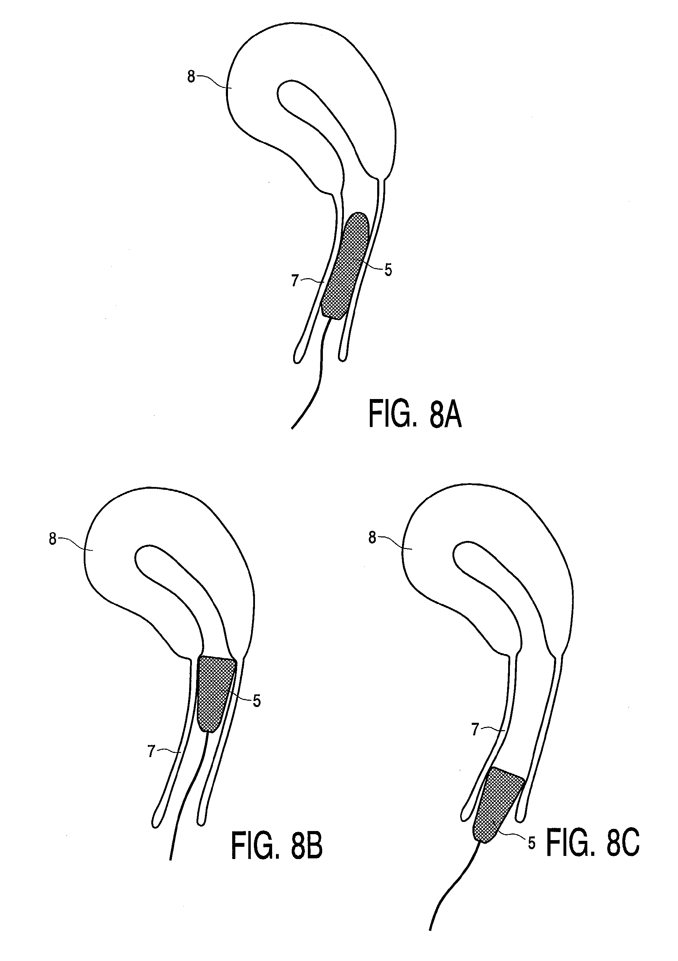

The insertion end 1 of a tampon 5 according to the invention is preferably, as is known per se, provided with a round dome, the outer edge of which is smoothed or chamfered. Such tampon according to the invention has the advantage of being easier to insert because the narrowed end goes deepest in the vagina. Placing this tampon within the vagina such that the insertion end 1 is near the cervix, i.e., toward the rear of the vagina, as is shown in **FIG. 8A**, is advantageous and desirable because this orientation promotes the most efficient wicking and wetting of a tampon considering that the origin of the fluid to be collected is deep in the vagina. Still a further advantage gained by orienting a tampon with a doom shaped insertion end 1 toward the rear of the vagina is that it faces a larger tampon surface. Thus, a larger surface is exposed to the natural secretion of the vagina, i.e., menses, mucus, and clottable whole blood, which otherwise could block a smaller surface more easily and prevent further menses absorption.

As illustrated in **FIG. 1-4**, tampons 5 are provided which have an outer surface that is at least partially provided with longitudinal grooves 6. The outer surface of the tampon 5 is also at least partially provided with longitudinal ribs defined by longitudinal grooves 6. The longitudinal ribs can be straight or spirally or helically shaped in the axial direction between the insertion end 1 and the withdrawal end 3. Different embodiments of longitudinal grooves 6 are illustrated in **FIG. 1-4**. The number of longitudinal ribs can vary, for example depending on the diameter of the tampon and/or the type of absorption material. Preferably, there are between 4 and 12 ribs, more preferably there are between 6 and 12 ribs and even more preferably, at least about eight. While the present invention, like many known tampons, my have an even number of ribs, it is also possible to produce tampons according to the present invention with an odd number of ribs.

The grooves 6 enlarge the surface of the tampon 5 and can thus reduce the risk of leakage. They can bring about an enlargement of the surface of the tampon, which results in the absorption capacity and expansion capacity of the tampon being considerably improved. This improvement can result from any depth of the groove 6. However, it is preferred that the groove has a depth of at least about 1 mm. In a preferred embodiment the groove may have a depth of more than about 1 mm, preferably about 2 mm to about 6 mm. The grooves can be brought to the tampon in any manner well known to those of ordinary skill in the art.

In a further embodiment, the withdrawal end 3 is provided with a finger recess, which is formed in the longitudinal direction of the tampon. This facilitates the handling and the insertion of the tampon 5.

Referring to **FIG. 1-4**, it is illustrated that a withdrawal cord 4 is attached to said tampon 5 and extends from the withdrawal end 3. The withdrawal cord 4 having a last point of attachment to the tampon is preferably flexible, hydrophobic, and strong enough in tension to resist breaking during removal of the product. It can be made of any of the materials used for withdrawal cord and well known to those of ordinary skill in the tampon art. It should be long enough. It can be a single cord, a tape, or a plurality of strings. Materials, which have worked well as the withdrawal cord, are a hydrophobic cotton string, a hydrophobic polyester string or a mixture hereof. Polyester makes the string stronger. The withdrawal cord 4 can be secured to the tampon 5 in any manner well known to those of ordinary skill in the art.

A tampon 5 according to the present invention may have the following dimensions and sizes. In a preferred embodiment, the invention provides a tampon having a longitudinal body 2 which preferably has a cross-sectional diameter comprised between 0.5 and 2.5 cm, and preferably between 1 and 2 cm. The longitudinal body 2, which is provided with an insertion end 1 and the withdrawal end 3, preferably has a length that is considerably greater than the length of the insertion end 1 or the length of the withdrawal end 3. In a preferred embodiment, the longitudinal body 2 is between two and five times longer than the insertion end 1 or the withdrawal end 3. The longitudinal body 2 may have a length comprised between 2 and 7 cm. The insertion end 1 and the withdrawal end 3 may each have a length comprised between 0.5 and 2.5 cm. In a preferred embodiment, the insertion end 1 and the withdrawal end 3 essentially have approximately a same length.

In a preferred embodiment, the tampon 5 is provided with a constricted withdrawal end 3. In a particularly preferred embodiment the withdrawal end 3 has a frusto-conical shape, as illustrated on **FIG. 1**. This shape substantially facilitates withdrawal of the tampon for the user. In addition, the invention provides a tampon 5 which has a reduced expansion capacity in the withdrawal end 3 compared to the remaining tampon body 2. This is desirable, in order to facilitate withdrawal. Less expansion of the withdrawal end 3, and consequently, less absorptive capacity, will also prevent leakage of body fluid into the withdrawal cord 4. Various technical features may be applied according to the invention to reduce the expansion and the absorptive capacity in the withdrawal end 3 of the present tampon.

According to the present invention a frusto-conical shape of the withdrawal end 3 and reduced expansion capacity in the withdrawal end 3 of the tampon 5 can be obtained in different ways. In an embodiment, the tampon 5 is more compressed at the withdrawal end 3 than at the remaining tampon body 2. In another embodiment of the invention, the tampon 5 is uniformly compressed at the withdrawal end 3 and at the remaining tampon body, but a higher amount of absorbent material per length unit is provided at the withdrawal end 3.

Expansion of the withdrawal end 3 can also be reduced by lowering the absorption potential on the material provided at withdrawal end 3, e.g. by using less amounts of high absorption fibre per length unit at the withdrawal end 3.

Another possibility for preventing expansion of the withdrawal end 3 consists of providing a covering on the circumferential surface of the withdrawal end 3. A covering having reduced liquid permeability or even a hydrophobic covering can be applied in, or in the vicinity of, the withdrawal end 3 in order to prevent absorption and expansion of the withdrawal end 3.

In addition, in another preferred embodiment, expansion of the withdrawal end 3 is reduced by providing expansion prevention means in the withdrawal end 3. Such means may comprise, a non elastic band, like non elastic polymeric films, for example polypropyleen, polyester, non elastic non woven webs, non elastic mono-layered non woven webs, non elastic non woven laminates.

In a further preferred embodiment, the present invention provides a tampon 5 having a lower expansion capacity in the withdrawal end 3 compared to the remaining tampon body 2. The present invention also provides a tampon having a higher expansion capacity in the insertion end 1 than in the remaining tampon body 2. Several technical features, which improve the expansion capacity in the insertion end 1 of the present tampon, are described in more detail hereafter.

In a preferred embodiment, a higher expansion capacity in the insertion end 1 of the tampon is obtained by providing a tampon 5 wherein the insertion end 1, and possibly also the area of the tampon body 2 which is nearest the insertion end 1, of said tampon 5 has a lower density than the remainder of the longitudinal body 2 of said tampon, so that a differentiated expansion of the tampon while absorbing fluid is obtained. A lower density of the insertion end 1 of the tampon 5 will allow the tampon to expand more at the insertion end 1 than in the remaining body 2 of the tampon. This effect facilitates withdrawal of the tampon, especially when menses fluid is limited or the cavity is relatively dry. In another embodiment, the tampon is uniformly compressed at the insertion end 1 and at the remaining tampon body 2, but a lower amount of absorbent material per length unit is provided at the insertion end 1.

A further advantage of this embodiment is that higher expansion capacity in the insertion end 1 of the tampon induces faster absorption and facilitates liquid uptake in this area of the tampon. The insertion end 1 will therefore be able to absorb menses faster and thus in practice to absorb more menses.

The tampon has a compressed, central, solid, generally cylindrical fibre core of highly compressed fibrous material, which ensures the stability or column strength of the tampon during digital introduction of the tampon into a body cavity. The longitudinal ribs are at least partially relatively uncompressed compared with the fibre core, and preferably have, in particular on a circumferential surface of the tampon, a soft fibrous structure. The longitudinal ribs that are defined by said longitudinal grooves 6 extend outward said fibre core. The longitudinal ribs 6 extend outward at equal circumferential angle intervals from this solid fibre core between the insertion end 1 and the withdrawal end 3. According to a further embodiment of the invention, the circumferential surface of the tampon and its fibre core can be substantially cylindrical with a circular cross-section, or even an oval cross-section.

In another embodiment, various perforations are provided on particular areas of the tampon. Preferably perforations are provided in the insertion end 1 of the tampon and or in the tampon body 2 nearest the insertion end 1, so that the tampon will expand more and absorb more in or near the insertion end 1 of the tampon than in the remaining part of the tampon body 2 when body fluid is absorbed.

In a preferred embodiment, perforations are provided in the covering of the tampon body 2 nearest the insertion end 1 of the tampon. The covering is preferably made of a liquid permeable film. The covering can consist of, for example, a non woven covering material made of, for example, thermoplastic, heat sealing fibers or a plastic film, of which the perforations are fluid pervious. The number and/or the size of the perforations define the absorption velocity or rate, as described in the prior art, for example in US 2001/0014348, which is incorporated herein by reference.

In another preferred embodiment, perforations are preferably also provided in the fibre material of the tampon, and preferably in the tampon body 2 nearest the insertion end 1 of the tampon, in order to improve absorption and expansion capacity of the tampon in this area. More in particular, the material in this area is at least partially perforated.

The invention provides a tampon having an insertion end 1 provided with a round dome. In a preferred embodiment, said round dome has various perforations. The dome is preferably not covered and perforations are thus applied directly in the fibre material of this dome.

The perforations in the insertion end 1 and in the covering and or fibre material of the tampon body 2 nearest the insertion end 1 may consist of holes of any form, for example, having a round, a triangular, a rectangular and or an ellipse cross-section. These holes may have non-uniform and various dimensions. By choosing the geometrical configuration of the perforations, it is possible to vary the size of the openings of the perforations whilst the observer would not notice whether the holes are in fact larger or smaller, as described in the prior art, for example in US 2001/0014348, which is incorporated herein by reference.

Perforations may have non-uniform and various dimensions with small diameters of the perforations and/or holes in the insertion end 1 of the tampon and or in the tampon body 2 nearest the insertion end 1. This provides for an optimal diversification of the absorption velocity and the absorption capacity in this area. The tampon can also be configured in such a manner that the absorbance characteristics of the tampon can be improved by the shape taken by the expanded tampon.

The degree of perforation can affect the absorbency, particularly the expansion rate of the tampon, and can also affect the expansion of a special area of the tampon. It is possible to control the different absorbent characteristics and a different absorption capacity in dependence on the number and/or size of the perforations in the insertion end 1 of the tampon and or in the tampon body 2 nearest the insertion end 1. The percent of open area as defined by the degree of the perforations in this area can be modified in a different manner. It is possible to modify the number and/or the distance of the perforations or to modify the size of the openings of the perforations. It is understandable and may be preferable to combine both of the aforementioned modifications.

For further improving the absorptive capacity of a tampon according to the present invention, a liquid absorptive covering can surround the tampon. In a preferred embodiment, the invention provides a tampon, wherein said tampon is at least partially surrounded by a covering. More in particular, said covering preferably extends over the longitudinal body 2. The covering is thus preferably not provided over the insertion end 1, in order to provide better access of the menses to the insertion end 1 of the tampon. In order to improve the absorbing capacity and expansion capacity of the tampon in the tampon body 2, and especially in the part nearest to the insertion end 1, said covering is preferably a stretchable or elastic liquid-permeable covering. The covering can consist of, for example, a non woven covering material made of, for example, thermoplastic, heat sealing fibers or a plastic film. Such a covering improves the comfort of introduction and prevents fibres being detached during introduction or removal of the tampon into or from the body cavity.

In another preferred embodiment, the withdrawal end 3 of the tampon 5 is provided with a hydrophobic covering, in order to limit absorption in the withdrawal end 3 and expansion of the withdrawal end 3.

In another preferred embodiment, a tampon according to the invention is provided, wherein the insertion end 1 and optionally also the part of the tampon body 2 which is nearest the insertion end 1 are provided with one or more hydrophilic components.

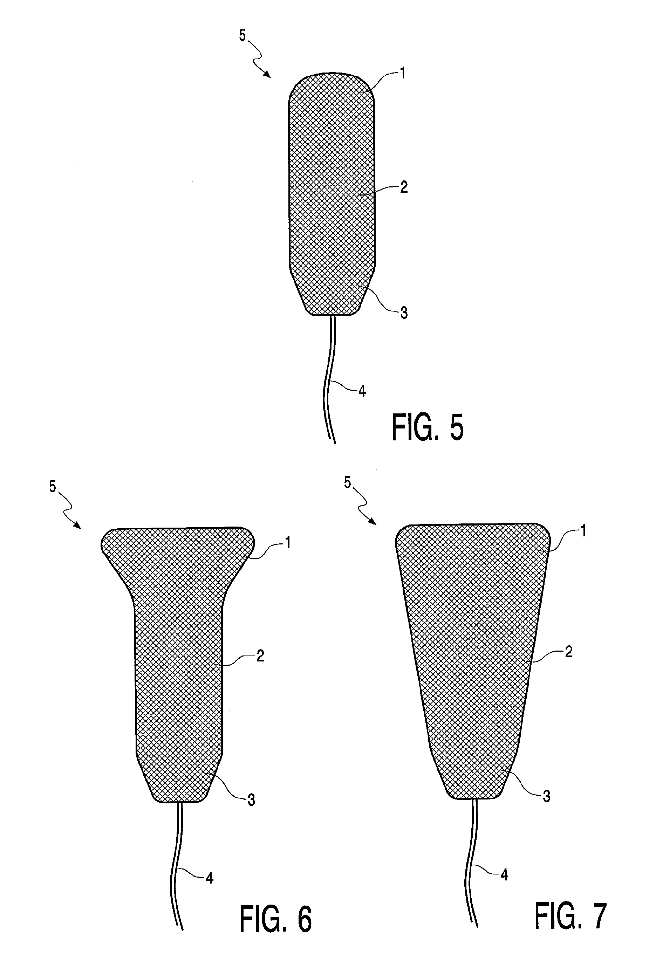

A tampon according to the invention is flexible and resilient. It deforms and expands under the pressures exerted by the vagina and the absorbed fluid. **FIG. 5** illustrates a tampon 5 according to the present invention before use, while **FIG. 6 and 7** illustrate tampons 5 according to the invention which have absorbed menses fluid and have expanded. In expansion, a tampon 5 may take the shape as shown in **FIG. 6 or 7**. The absorption characteristics are controlled by but not limited to: the lower material density provided in the insertion end 1; the size and the position of the perforation holes, hydrophilic substances in the insertion end 1 and or in the part of the tampon body 2 which is nearest the insertion end 1; a withdrawal end 3 with lower expansion capacity and or surrounded by a non stretchable cover material; or any combination thereof. The diameter of the expanded tampon thus increases in the direction of its insertion end 1, as illustrated on **FIG 6 and 7**.

**FIG. 8** illustrates the use of a tampon according to the invention. In **FIG. 8A** the tampon 5 is inserted in the vagina 7. The tampon 5 illustrated in **FIG. 8A** has a bullet shape at the insertion end 1 and a frusto-conical shape at the withdrawal end 3 when introduced in a vagina 7. Because the tampon according to the invention has a dome shaped insertion end 1, the tampon 5 is inserted deeply in the vagina 14, preferably such that the insertion end 1 is near the cervix 8. This orientation promotes the most efficient wicking and wetting of a tampon 5 considering that the origin of the fluid to be collected is deep in the vagina 7. By absorbing menses, the tampon 5 may expand as illustrated in **FIG. 8B**. When, according to the invention, expansion and absorption capacity of the tampon are considerably increased at and or near the insertion end 1 of the tampon compared to the remaining tampon body 2, the tampon diameter will decrease towards the withdrawal end 3. Since the tampon 5 can be deeply introduced in the vagina 7, the largest diameter is provided near the origin of the menses, i.e., the cervix 8, where the menses are most concentrated and released most heavily. **FIG. 8C** illustrates withdrawal of the tampon from the vagina 7. When removing the tampon 5 from the vagina 7, the tampon 5 proceeds with its narrowest end first, and the expanded insertion end 1 acts somewhat like a wedge. The wedge action gently urges the vagina 7 to distend. This urging action is beneficial in the introital region where the sphincter surrounding the vagina 7 acts to keep the vagina 7 closed. The removal comfort is enhanced if the sphincter is gradually distended, rather than suddenly distended, and this gradual distension is accomplished by a wedge shaped tampon because the tampon cross section is gradually increased.

Absorption by the withdrawal cord 4 can result in stains in underpants. This is particularly undesirable from a hygienical and esthetical point of view. By improving the absorption capacity of the tampon 5, the risk that menses are absorbed by the withdrawal cord 4 can be limited.

The invention also provides a method for producing a tampon according to the invention. In general, the method includes providing a tampon blank of tangled fibrous material; and compressing the tampon blank whereby longitudinal grooves 6 are at least partially formed at the outer surface of the tampon, in order to enlarge the absorbent surface of the tampon. In a preferred embodiment the method comprises the steps of providing a tampon blank of tangled fibrous material having a longitudinal direction; and compressing the tampon blank whereby longitudinal grooves and longitudinal ribs are at least partially formed at an outer surface of the tampon, and whereby the fibrous material in the area of at least the insertion end and the withdrawal end of the tampon blank is subjected to a higher radial compressing pressure than the remaining fibrous material of the tampon blank. In order to obtain a higher density of fibrous material in the withdrawal end, the tampon blank should additionally either contain more material in the area of the withdrawal end or the material in the withdrawal end should be subject to a higher compression than the insertion end. The tampon blank is radially compressed with respect to the longitudinal direction. The tampon blank can be compressed to provide a fibre core with a high degree of compression, from which relatively uncompressed longitudinal ribs extend radially outward.

Preferably, the method comprises providing the insertion end 1 of the tampon 5 with a round dome chamfered off at the outer edge. By applying a higher radial compressing pressure at the insertion end and the withdrawal end of the tampon blank, a tampon is obtained having a round dome at the insertion end 1 and a frusto-conical shaped withdrawal end 3.

In a preferred embodiment the invention relates to a method, wherein the tampon blank is produced from a section of a non woven strip which is wound up on itself. In order to provide the tampon with a withdrawal cord 4, a withdrawal cord is wrapped around the section of non woven strip, before it is wound up into the tampon blank. In another preferred embodiment, the invention relates to a method, wherein the withdrawal end 3 of the tampon is provided with a finger recess.

In order to provide grooves 6 in the tampon, the method comprises compressing the tampon blank on its outer circumferential surface, forming straight longitudinal grooves and a fibre core with a high degree of compression from which relatively uncompressed longitudinal ribs extend radially outward. Alternatively, the method comprises compressing the tampon blank on spiral lines of its outer circumferential surface, forming spiral longitudinal grooves and a fibre core with a high degree of compression from which relatively uncompressed spiral longitudinal ribs extend radially outward. Preferably, the tampon blank is compressed such that said (straight or spiral) longitudinal ribs extend radially outward at equal circumferential angle intervals and between the insertion end and the withdrawal end.

In order to provide the tampon with a covering sheath, the method further comprises the step of applying a strip of a covering and fixing said strip at least to the end of that side of the non woven section that lies on the outside during the manufacture of the tampon before the non woven section is wound up to form the tampon blank. The covering at least partly covers the circumferential surface of the tampon blank, and preferably does not cover the round dome, which is chamfered off at the insertion end.

In detail, a preferably cylindrical tampon blank is introduced in a press apparatus. A preferred press apparatus comprises a press having press jaws which are arranged in a star formation with respect to the press axis and can be moved synchronously in a common plane radially with respect to the press axis between their open position and closed position and, in their closed position, are supported on one another on their mutually opposite longitudinal sides. A preferred press consists of eight press jaws which are arranged in a star formation in a common plane at equal angle intervals about and at the same radial distance from the press axis. It is desirable to equip the press with an even number of press jaws, but other numbers of press jaws can be used, including odd numbers. The number of press jaws can vary, for example depending on the weight and the composition of the material intended for the tampon and can also be smaller or greater than eight, although the number generally should not be under four. The press jaws can be moved synchronously radially with respect to the press axis between their open position and closed position.

The press jaws can preferably be heated and preferably each press jaw has its own temperature sensor. By heating the press jaws, it is possible to reduce the memory effect of modern, highly absorbent, greatly expanding fibrous materials, which occurs after the tampon has been finished. By means of the heated press jaws, the surface of the tampon is simultaneously smoothed during pressing and pushing out, and a qualitatively improved surface is produced even in tampons of low weight, the stability of the tampon being preserved. The memory effect of the fibrous material becomes effective again when the fibrous material of the tampon is wetted with body fluid. Each press jaw preferably has a curvature from one end of the press jaw to the other end of the same press jaw. This curvature ensues from the fact that the effective pressing surface of each press jaw has to press a (straight or spiral) longitudinal groove.

From the above description of the press jaws it is preferred that the press jaws lie diametrically opposite one another in pairs. The present invention also includes the possibility of, in addition to the press jaws described, which produce the fibre core, building press jaws into the press, which serve purposes other than the production of the fibre core. Accordingly, it is possible to use press jaws within the press in order, for example, to stamp patterns or depressions onto or into the surface of the tampon during pressing of the preform, which are intended to serve decorative and/or physical purposes.

The tampon blank can be made of a length of a fibrous non woven material, at the end of which a sheathing material is fixed on the outside. The tampon blank is wound up to form a tampon blank, which is subsequently radially compressed by press jaws, such as those described above. Preferably the tampon blank is pressed over identical, narrow, sections of its circumferential surface. In this way, a preform is produced, with longitudinal grooves on a solid fibre core with a high degree of compression, which is substantially cylindrical. In the press, the fibrous material is preferably subjected to higher radial compressing pressure in the area of the insertion end and the withdrawal end of the tampon blank than the remaining fibrous material of the tampon blank. In order to obtain a higher density of fibrous material in the withdrawal end, the tampon blank should additionally either contain more material in the area of the withdrawal end or the material in the withdrawal end should be subject to a higher compression than the insertion end. The fibre core is thus reduced in cross section at both ends of the tampon.

The tampon blank is, depending on the properties of the fibrous material used, in particular in the event of use being made of highly expansive fibres of irregular cross section with a strong memory effect, pressed at a temperature of the press jaws to the final shape of the tampon, in order to achieve the desired dimensional stability of the fibrous material by eliminating the memory effect of the fibres, which immediately becomes effective again on contact with bodily fluid and thus increases the expansion and absorption speed of the tampon with the least possible use of fibrous material.

In the press, the tampon blank is preferably compressed in a single pressing operation to form the preform, which, on ejection from the press, may be at the same time subjected to final shaping downstream. This final shaping may include a weak radial pressure being exerted on the outer ends of the longitudinal ribs and on the longitudinal grooves. This weak radial pressure has the effect that the outer ends of the mutually opposite side flanks of adjacent longitudinal ribs are pressed against one another so that the longitudinal grooves and thus the outer, approximately cylindrical, soft, closed circumferential surface of the tampon are shaped, and liquid guide ducts are produced in the area of the now radially outwardly closed longitudinal grooves, which ducts are preferably open at the insertion end and at the withdrawal end of the tampon. In this way, a considerable increase in the absorption capacity of the tampon is achieved along with very comfortable introduction for the user.

It is apparent that there has been provided in accordance with the invention, a tampon that fully satisfies the objects, aims, and advantages set forth above. While the invention has been described in conjunction with specific embodiments thereof, it is evident that many alternatives, modifications, and variations will be apparent to those skilled in the art in light of the foregoing description. Accordingly, it is intended to embrace all such alternatives, modifications, and variations as follows in the spirit and broad scope of the appended claims.

## Claims

1. A tampon (5) for feminine hygiene having a longitudinal body (2) provided with a round domed insertion end (1) and a withdrawal end (3), from which withdrawal end (3) a withdrawal cord (4) extends, whereby the tampon (5) essentially consists of compressed absorbent fibrous material and optionally comprises longitudinal grooves (6), whereby said tampon (5) comprises differentiated expansion capacity in the longitudinal direction, decreasing towards the withdrawal area.

2. A tampon (5) for feminine hygiene having a longitudinal body (2) provided with a round domed insertion end (1) and a withdrawal end (3), from which withdrawal end (3) a withdrawal cord (4) extends, and whereby the tampon (5) essentially consists of compressed absorbent fibrous material and whereby said tampon (5) comprises longitudinal grooves (6) and whereby said tampon has a constricted withdrawal end.

3. Tampon according to claim 1 or 2, wherein said withdrawal end (3) is provided with a finger recess.

4. Tampon according to any of claims 1-3, wherein said withdrawal end (3) has a frusto-conical shape.

5. Tampon according to any of claims 1-4, wherein said tampon (5) has a reduced expansion capacity in the withdrawal area compared to the remaining tampon body.

6. Tampon according to any of claims 1-5, wherein the withdrawal area of said tampon (5) is provided with a covering which is not stretchable nor elastic.

7. Tampon according to any of claims1-6, wherein said tampon (5) is uniformly compressed at the withdrawal end (3) and at the remaining tampon body, and wherein a higher amount of absorbent material per length unit is provided at the withdrawal end (3).

8. Tampon according to any of claims 1-7, said tampon (5) having a higher expansion capacity in the top than in the remaining tampon body.

9. Tampon according to any of claims 1-8, wherein the insertion end (1) of said tampon has a lower density than the longitudinal body (2) of said tampon.

10. Tampon according to any of claims 1-9, wherein said tampon (5) is uniformly compressed at the insertion end (1) and at the remaining tampon body, and wherein a lower amount of absorbent material per length unit is provided at the insertion end (1).

11. Tampon according to any of claims 1-10, wherein the tampon (5) is compressed radially with respect to the longitudinal direction.

12. A tampon according to any of claims 1-11, wherein said tampon (5) is provided with 6 to 12 longitudinal grooves (6).

13. Tampon according to any of claims 1-12, wherein said longitudinal grooves (6) are spirally shaped.

14. Tampon according to any of claims 1-13 wherein the tampon (5) has a fibre core of highly compressed fibrous material from which fibre core longitudinal ribs extend radially outward that are defined by said longitudinal grooves (6).

15. Tampon according to any of claims 1-14, wherein said longitudinal ribs are at least partially relatively uncompressed compared with the fibre core.

16. Tampon according to any of claims 1-15 wherein said longitudinal ribs extend radially outward at equal circumferential angle intervals and between the insertion end (1) and the withdrawal end (2).

17. Tampon according to any of claims 1-16, wherein perforations are provided in the covering in the top area of the tampon.

18. Tampon according to any of claims 1-17, wherein the fibrous material provided at the upper part of said tampon is at least partially perforated.

19. Tampon according to any of claims 1-18, wherein said round dome has various perforations.

20. Tampon according to any of the claims 1-19, wherein said withdrawal end (3) is provided with a hydrophobic covering.

21. Tampon according to any of claims 1-20, wherein the top of the tampon insertion end (1) is provided with one or more hydrophilic components.
